# FASCICULE DE BREVET EUROPEEN

(11) **EP 1 053 277 B1**
(45) Date de publication et mention de la délivrance du brevet: **22.01.2003**
(21) Numéro de dépôt: 99903747.6
(22) Date de dépôt: 12.02.1999
(51) Int. Cl.: C08J 3/21, A61K 7/00

(54) **PARTICULES COMPOSITES CONTENANT UNE MATIERE ACTIVE**
EIN AKTIVES MATERIAL ENTHALTENDE VERBUNDPARTIKEL
COMPOSITE PARTICLES CONTAINING AN ACTIVE SUBSTANCE

(30) Priorité: 13.02.1998 FR 9801780
(43) Date de publication de la demande: 22.11.2000
(73) Titulaire: RHODIA CHIMIE, 92408 Courbevoie Cédex (FR)
(72) Inventeur: DUPUIS, Dominique, 95170 Deuil-la-Barre (FR); REEB, Roland, F-77410 Gressy (FR)
(74) Mandataire: Le Coupanec, Pascale
(86) Numéro de dépôt international: FR9900321
(87) Numéro de publication internationale: WO99041298

(56) Documents cités:
- EP-A- 0 211 298
- WO-A-97/32920
- FR-A- 2 528 700
- US-A- 5 372 804

## Description

La présente invention a pour objet des particules composites comprenant un coeur à base d'au moins un polymère organique dans lequel est dispersée au moins une matière organique hydrophobe et d'un revêtement externe inorganique.

Plus précisément, la présente invention vise la mise au point d'un système d'encapsulation approprié au conditionnement d'une matière active hydrophobe et à son relargage de manière contrôlée.

Des systèmes d'encapsulation pour des matières actives ont déjà été développés. A titre illustratif de ceux-ci, on peut tout particulièrement mentionner ceux mettant en oeuvre des matériaux particulaires polymériques à titre de support pour matière(s) active(s). Il peut notamment s'agir de microparticules de latex dans lesquelles sont dispersées lesdites matières actives.

Toutefois, ce mode d'encapsulation n'est pas complètement satisfaisant dans la mesure où il ne permet pas de garantir une totale protection à la matière active qu'il contient vis à vis du milieu extérieur et réciproquement. Le fait que la matière active soit dispersée de manière uniforme au sein des particules implique que cette matière active soit présente au coeur de la particule mais également en sa périphérie. En ce qui concerne plus particulièrement la matière active présente en périphérie, il est clair qu'elle est exposée au moins en partie au milieu extérieur. Elle demeure donc vulnérable à toute variation physico-chimique de ce milieu telle par exemple modification de pH, température ou luminosité. Il est évident que ceci peut se traduire par des transformations chimiques au niveau de la matière active accessible et/ou contribuer à sa diffusion hors de la particule.

En conséquence, ce type d'encapsulation n'est pas totalement satisfaisant en terme de protection de la matière active ni du contrôle de son relargage.

L'objectif de la présente invention est précisément de proposer un nouveau système d'encapsulation permettant de s'affranchir des problèmes évoqués précédemment.

Elle vise à proposer des particules composites comprenant un coeur organique dans lequel est dispersée la ou les matière(s) active(s), ce coeur étant isolé du milieu extérieur par un enrobage de nature inorganique.

Cet enrobage ou encore écorce permet avantageusement de répondre aux exigences évoquées ci-dessus. Il garantit une protection à la matière active encapsulée dans le coeur organique vis à vis du milieu extérieur et permet, le cas échéant, de contrôler sa diffusion grâce au choix spécifique des composants de cette écorce inorganique.

La présente invention a donc pour premier objet des particules composites comprenant au moins
- un coeur composé d'au moins un polymère organique dans lequel est dispersé au moins une matière active hydrophobe, cosmétique, pharmaceutique, alimentaire ou phytosanitaire
- un revêtement externe comprenant au moins un oxyde et/ou hydroxyde d'aluminium, de silicium, de zirconium et/ou d'un métal de transition et
- une couche intermédiaire, présente au moins en partie entre le coeur et le revêtement externe et comprenant au moins un hydroxyde de métal alcalino-terreux.

Un autre objet de l'invention est constitué par un procédé utile pour la préparation des particules composites précitées.

Les particules composites revendiquées possèdent donc avantageusement une écorce de nature inorganique. Cette écorce inorganique est constituée d'au moins deux couches superposées, une couche dite intermédiaire et une couche dite revêtement externe. La couche intermédiaire a pour fonction principale de consolider l'adhésion entre le coeur de nature inorganique et la couche la plus externe de nature inorganique.

Le revêtement (ou indifféremment, l'écorce de la couche externe) à base d'oxyde et/ou d'hydroxyde peut recouvrir en partie seulement ou en totalité, chaque coeur de polymère organique. Il est également possible que ce revêtement soit en partie incrusté dans la couche intermédiaire recouvrant le coeur en polymère organique voire dans le coeur organique lorsque celui ci n'est que partiellement recouvert par la couche intermédiaire.

La nature des polymères organiques constituant le coeur des particules composites est de préférence du type de celle des particules de latex, c'est-à-dire des particules de (co)polymères issues de procédés classiques de (co)polymérisation en émulsion de monomères organiques copolymérisables.

Plus généralement, ce polymère dérive d'au moins un monomère éthyléniquement insaturé non miscible à l'eau et polymérisable par voie radicalaire.

A titre illustratif des monomères convenant à l'invention, on peut notamment citer les monomères vinylaromatiques, les alkylesters d'acides α-β éthyléniquement insaturés, les esters de vinyle d'acides carboxyliques, les halogénures de vinyle ou de vinylidène, les diènes aliphatiques conjugués et les nitriles α-β éthyléniquement insaturés.

A titre illustratif de ce type de monomères on peut notamment citer le styrène, butadiène, acrylonitrile, chloroprène, chlorure de vinyle ou de vinylidène, isoprène, isobutylène, acétate de vinyle, propylène, butylène et la vinylpyrrolidone.

Selon un mode particulier de l'invention, il peut s'agir d'un polymère réticulé. Cette réticulation est obtenue par incorporation d'un agent réticulant comme le divinylbenzène, le diméthacrylate élhylène glycol (DIMEG), le méthacrylate d'allyle, le méthylène bisacrylamide, lors de la synthèse dudit polymère. Le choix de l'agent réticulant est opéré en fonction de la nature des monomères considérés.

Le polymère constituant le coeur des particules composites selon l'invention peut également comprendre jusqu'à 8 %, de préférence jusqu'à 4 % d'au moins un co-monomère portant des groupes ionogènes polymérisables par voie radicalaire. Il peut notamment s'agir d'un monomère choisi parmi les acides carboxyliques α-β éthyléniquement insaturés, les monomères éthyléniques sulfonés, les hydroxyalkylesters d'acides α-β éthyléniquement insaturés d'hydroxypropyle, les amides d'acides α-β éthyléniquement insaturés et les aminoesters d'acides α-β éthyléniquement insaturés.

A titre d'exemples de ces groupes ionogènes on peut tout particulièrement mentionner ceux choisis parmi les acides acrylique, méthacrylique, itaconique, maléique, crotonique, para-styrènesulfonique, vinylsulfonique, 2-méthacryloyloxyéthylsulfonique, 2-acrylamido-2-méthylpropylsulfonique, vinylbenzène sulfonate, acrylates et méthacrylates d'hydroxyéthyle, acrylamide, méthacrylamide et diéthylaminoéthylméthacrylate.

D'une manière préférentielle, le polymère ne comporte pas de monomères à fonctions carboxyliques ou sulfoniques.

Plus préférentiellement, ce polymère est choisi parmi les polystyrène, copolymères de styrène et d'esters acryliques, de styrène et de butadiène, de styrène, de butadiène et d'acrylamide.

On peut mentionner plus particulièrement les copolymères du styrène avec les acrylates ou le butadiène. Ils pourront avantageusement être choisis parmi les copolymères butadiène-styrène, les copolymères acryliques et les copolymères butadiène - styrène - acrylamide.

Ces polymères organiques présentent plus particulièrement une température de transition vitreuse comprise entre -100 °C et 150 °C. Selon un mode de réalisation particulièrement avantageux et préféré de l'invention, la température de transition vitreuse est comprise entre -100 °C et 100 °C et plus préférentiellement entre -15 °C et 100 °C.

Les polymères pouvant être mis en oeuvre selon l'invention possèdent une taille particulaire comprise entre 0,02 et 5 µm et de préférence entre 0,19 et 0,3 µm.

De manière générale, le diamètre du coeur en polymère organique incorporant la matière active peut varier entre environ 0,03 et 10 µm. Plus préférentiellement, il est de l'ordre de 0,1 à 1 µm.

En ce qui concerne le revêtement externe des particules composites revendiquées, il est constituée d'au moins un oxyde et/ou hydroxyde d'aluminium, de silicium, de zirconium, et/ou d'un métal de transition.

Par métal de transition, on entend plus particulièrement les métaux de la quatrième période, allant du scandium au zinc dans la mesure où ceux-ci sont compatibles en terme d'innocuité avec l'application visée. Il s'agit plus particulièrement d'un oxyde et/ou hydroxyde de titane, manganèse, fer, cobalt, nickel ou de cuivre.

Conviennent particulièrement bien à l'invention les oxydes et/ou hydroxydes de silicium, aluminium, titane et de zirconium.

Il est à noter que ce revêtement peut comprendre un oxyde et/ou un hydroxyde d'un seul ou de plusieurs éléments dans une même couche. En particulier, il peut s'agir d'oxydes mixtes comme par exemple un aluminosilicate.

Selon une première variante de la présente invention, le revêtement externe selon l'invention est composé d'une seule couche à base d'au moins un oxyde et/ou d'hydroxyde d'aluminium, de silicium, de zirconium, et/ou d'un métal de transition.

Selon une seconde variante de la présente invention, ce revêtement externe comprend au moins deux couches, à base d'au moins un oxyde et/ou hydroxyde d'aluminium, de silicium, de zirconium, et/ou d'un métal de transition. Dans un tel cas, les deux couches superposées, recouvrant au moins partiellement le coeur des particules composites, et se recouvrant elles-mêmes au moins partiellement, peuvent être à base d'un ou plusieurs oxydes et/ou hydroxydes tels qu'identifiés précédemment.

Comme cela a été indiqué auparavant, les particules composites selon l'invention présentent en outre une couche dite intermédiaire recouvrant au moins en partie les particules de polymère organique décrites ci-dessus.

Cette couche intermédiaire, permet avantageusement de renforcer la fixation du revêtement externe inorganique décrit ci-dessus à la surface du coeur organique.

Elle est généralement composée d'un ou plusieurs hydroxydes d'alcalino-terreux. Il peut notamment s'agir d'hydroxyde de calcium et/ou d'hydroxyde de magnésium.

L'épaisseur totale du revêtement appliqué en surface du coeur organique des particules revendiquées, c'est-à-dire comprenant une ou plusieurs couches à base d'au moins un oxyde et/ou hydroxyde d'aluminium, de silicium, de zirconium, et/ou d'un métal de transition et une couche d'au moins un hydroxyde de métal alcalino-terreux est généralement au plus de 300 nm. Elle est habituellement au moins de 1 nm. Plus particulièrement, elle est comprise entre 1 et 300 nm et de préférence entre 5 et 100 nm.

Les matières actives pouvant être incorporées au sein du polymère organique constituant le coeur des particules revendiquées sont des composés hydrophobes, cosmétiques, pharmaceutiques, alimentaires ou phytosanitaires.

Le système d'encapsulation particulaire mis au point selon l'invention est notamment particulièrement avantageux pour l'application d'agent(s) cosmétique(s) qui sont généralement très hydrophobes.

A titre illustratif de ces matières actives hydrophobes on peut notamment mentionner les agents organiques anti-UV, les huiles essentielles ou thérapeutiques, les parfums et les colorants cosmétiques.

Parmi les agents organiques anti-UV on peut tout particulièrement citer les composés de type benzophénone, les dérivés méthane dibenzoyle et cinnamate. Il peut notamment s'agir du 2-éthyl hexyl méthoxy cinnamate commercialisé à titre d'anti UV B sous le nom de Parsol MCX®.

La quantité en matière active dispersée au sein du polymère organique est bien entendu fonction de sa nature, du mode d'administration préconisé et du sujet traité.

A titre indicatif, cette matière active peut être dispersée au sein du polymère organique à raison de 0,1 à 200 % en poids exprimé par rapport au poids en polymère sec.

Par exemple, un agent anti-UV peut être dispersé dans le polymère organique à raison de 0,1 à 200 % en poids par rapport au poids en polymère sec.

Les particules composites selon l'invention présentent de préférence une surface spécifique comprise entre environ 1 et 200 m²/g, de préférence entre environ 1 et 100 m²/g.

On entend par surface spécifique, la surface spécifique BET déterminée par adsorption d'azote conformément à la norme ASTM D 3663-78 établie à partir de la méthode BRUNAUER - EMMETT - TELLER décrite dans le périodique "The Journal of the American Society", 60, 309 (1938).

Cette surface spécifique peut révéler l'aspect plus ou moins lisse du revêtement enrobant le polymère organique.

De manière générale, leur diamètre peut varier entre environ 0,02 et 6 µm et de préférence entre 0,1 et 0,4 µm.

La présente invention a pour second objet un procédé utile pour la préparation de particules composites conformes à la présente invention.

Plus précisément, le procédé revendiqué se caractérise en ce qu'il comprend au moins:
- l'incorporation d'au moins une matière active hydrophobe dans des particules à base d'au moins un polymère organique, lesdites particules étant en dispersion aqueuse, alcoolique ou hydroalcoolique et le cas échéant formées conjointement à l'incorporation de ladite matière active,
- la solubilisation d'au moins un sel de métal alcalino-terreux dans la phase liquide de la dispersion de particules de polymère et contenant au moins une matière active hydrophobe obtenues selon l'étape précédente,
- l'addition d'une base en quantité suffisante pour précipiter une couche d'oxyde ou d'hydroxyde dudit métal alcalino-terreux à la surface desdites particules,
- la mise en contact, dans la phase liquide de la dispersion de particules obtenue à l'issue de l'étape précédente, d'au moins un sel de silicium, aluminium, zirconium et/ou d'un métal de transition hydrosoluble et d'au moins un agent susceptible de réagir avec ledit sel soluble pour former un précipité constitué de l'oxyde ou de l'hydroxyde correspondant à la surface desdites particules et
- le séchage et la récupération des particules composites ainsi obtenues.

Comme évoqué ci-dessus, l'incorporation de la matière active au niveau de la dispersion aqueuse de particules à base d'au moins un polymère organique peut être réalisée selon deux variantes. La matière active peut être introduite soit avant le déroulement de la polymérisation radicalaire en émulsion des monomères ou à l'issue de cette polymérisation.

La polymérisation en soit des monomères dont dérive le polymère organique peut être réalisée de manière conventionnelle.

Les particules à base d'au moins un polymère organique sont obtenues par polymérisation radicalaire en émulsion aqueuse, alcoolique ou hydroalcoolique de monomères éthyléniquement insaturés non miscibles à l'eau et polymérisables.

De préférence, la polymérisation est conduite en émulsion dans l'eau.

A cet effet, on met en oeuvre au moins un émulsifiant. Celui-ci peut être soit anionique ou non ionique.

Pour ce qui est des émulsifiants anioniques pouvant convenir à la présente invention, on peut citer les sels d'acides gras ; les alkylsulfates, alkylsulfonates, alkytarylsulfonates, alkylsulfosuccinates, alkylphosphates alcalins ; les sulfosuccinates d'alkyle ; les sulfonates d'éthers alkylphénolpolyglycoliques ; les produits de condensation des acides gras avec les acides oxy- et amino- alcanesulfoniques ; les dérivés sulfatés des éthers polyglycoliques ; les esters sulfatés d'acides gras et de polyglycols ; les alcanolamides d'acides gras sulfatés.

Quant aux émulsifiants non ioniques, ils peuvent être choisis parmi : les esters gras de polyalcools, alcanolamides d'acides gras, polyoxydes d'éthylène, alcools et alkylphénols oxyéthylénés, alcools et alkylphénols oxyéthylénés et sulfatés.

Ces émulsifiants sont de préférence présents dans l'émulsion à raison d'environ 0,1 à 3 % en poids par rapport aux poids de monomères.

En ce qui concerne les monomères, il s'agit de monomères tels que décrits précédemment dans le cadre de la définition des particules composites revendiquées.

Ces monomères sont présents à raison de 5 à 55 % en poids dans l'émulsion aqueuse.

La réaction de polymérisation est conduite en présence d'un amorceur. Celui-ci est généralement incorporé à raison d'environ 0,05 à 4,5 % en poids et de préférence à raison de 0,1 à 2 % en poids des monomères. Bien entendu, l'amorceur considéré est soluble en milieu aqueux ou hydroalcoolique. A titre illustratif de ces amorceurs, on peut notamment citer ceux choisis parmi : l'eau oxygénée, les persulfates alcalins, dérivés diazoiques, l'hydroperoxyde de tertiobutyle et les systèmes Redox à base d'oxydant (eau oxygénée, peroxydes et hydroperoxydes organiques) et de réducteur (sulfites et bisulfites alcalins, aminés).

D'autres réactifs classiquement mis en oeuvre en polymérisation radicalaire peuvent également être considérés dans le cadre de la présente invention. Par exemple, il peut être introduit dans le mélange de polymérisation, un agent réticulant et/ou un agent limiteur de chaîne. Ce dernier peut être généralement présent jusqu'à 3 % et de préférence 1 % en poids des monomères. Cet agent limiteur de chaîne peut être un hydrocarbure halogéné, un alcool aliphatique en C₁-C₄ ou encore un mercaptan.

Les conditions opératoires pour conduire la polymérisation dépendent bien entendu directement de la nature des monomères considérés ainsi que de l'amorceur retenu. Toutefois, à titre indicatif, la polymérisation peut généralement être effectuée à une température comprise entre environ 0° C et 100 °C et de préférence entre environ 50 °C et 85 °C.

A l'issue de la polymérisation en émulsion radicalaire on obtient une dispersion aqueuse de particules organiques.

Selon la première variante, l'incorporation de la matière active est effectuée conjointement à la polymérisation radicalaire des monomères. A cet effet, la matière active hydrophobe est solubilisée dans les monomères, préliminairement à leur mise en émulsion aqueuse, alcoolique ou hydroalcoolique puis à leur polymérisation radicalaire.

Selon la seconde variante qui correspond au mode préféré de l'invention, la matière active hydrophobe est introduite à l'issue de la polymérisation radicalaire des monomères. Dans ce cas particulier, son incorporation au sein des particules organiques est effectuée par mise en contact desdites particules organiques en dispersion aqueuse, alcoolique ou hydroalcoolique avec ladite matière active, le cas échéant, solubilisée dans un solvant de transfert.

Au sens de l'invention, un solvant de transfert désigne un solvant capable de promouvoir l'adsorption de la matière active qu'il contient par les particules composites de polymère organique. Cette adsorption peut notamment se traduire par un phénomène de gonflement desdites particules sous l'effet de leur perméabilité à l'égard du solvant de transfert.

Comme solvant de transfert susceptibles d'être mis en oeuvre selon l'invention, on peut notamment citer : les dérivés aromatiques (toluène, éthylbenzène), dérivés aromatiques chlorés (trichlorobenzène), hydrocarbures aliphatiques et cycliques (heptane, dodécane, cyclohexane, décaline...), dialkyléthers, alcools (propanol, pentanol, cyclohexanol...), esters (propionate de méthyle, acétate d'éthyle, mélange de glutamate/adipate/succinate de méthyle "RPDE"), cétones (méthyléthylcétone, cyclohexanone) dérivés chlorés aliphatiques (dichlorométhane) et les silicones.

Bien entendu le choix du solvant de transfert doit être effectué en considérant la nature de la matière active à solubiliser et la nature du polymère à perméabiliser.

C'est ainsi que dans certaines circonstances, la présence d'un tel agent de transfert peut s'avérer non nécessaire. En effet, certaines matières actives peuvent posséder de par elles-mêmes la faculté de pénétrer dans lesdites particules.

Pour favoriser le gonflement desdites particules, il peut être procédé à un chauffage du milieu réactionnel. Le choix de la température est directement lié à la nature du polymère constituant les particules. De préférence, on privilégie une température supérieure à la température de transition vitreuse du polymère pour favoriser l'expansion des chaînes polymériques. Dans le cas particulier, où on ne met pas en oeuvre d'agent de transfert il est préférable en terme de rendement d'incorporation, de procéder à un ajout en continu de la matière active au sein du milieu réactionnel.

De préférence, la mise en contact entre la matière active, en solution ou non dans un solvant de transfert, et les particules de polymères en suspension, est réalisée sous agitation et à une température comprise entre environ 20 et 90 °C.

L'adsorption de la matière active par les particules de polymère est considérée achevée au terme de 5 à 6 heures. L'augmentation de la taille des particules de polymères reflète le taux d'incorporation.

A l'issue de la première étape du procédé et ce quelque soit la variante d'adsorption retenue, on obtient des particules à base d'un polymère organique contenant une matière active hydrophobe et dont le taux d'extrait sec peut varier entre 5 et 70 %.

Les particules organiques de matières actives présentent généralement une taille comprise entre 0,03 et 10 µm et de préférence entre 0,1 et 1 µm.

A la fin de cette première étape, on peut le cas échéant procéder à l'élimination du solvant de transfert préalablement à l'ajout du ou des sel(s) de métaux alcalino-terreux dont dérive la couche intermédiaire.

Ce solvant peut être éliminé par toute méthode conventionnelle dans la mesure où celle-ci n'est pas de nature à affecter la stabilité de particules de polymères. On peut par exemple effectuer une évaporation sous vide dudit solvant de transfert. Toutefois, cette étape d'élimination peut dans certains cas ne pas s'avérer nécessaire.

En ce qui concerne la stabilité de la dispersion de particules de polymère incorporant la matière active hydrophobe, il peut le cas échéant être avantageux de la renforcer pour notamment prévenir tout risque de floculation au cours des étapes suivantes.

A cet effet, il peut être ajouté à la dispersion aqueuse, alcoolique ou hydroalcoolique obtenue à l'issue de la première étape du procédé revendiqué et avant sa mise en contact avec le ou les sel(s) soluble(s) de métaux alcalino-terreux, un ou plusieurs agent(s) stabilisant(s) de type alkylphénol polyéthoxylé, polyéthylène glycol ou polyvinylpyrrolidone.

Ce stabilisant peut être incorporé à raison d'environ 0,1 à 5 % et de préférence jusqu'à 2 % en poids par rapport au poids de matières sèches de ladite dispersion.

La dispersion de particules organiques ainsi obtenue est ensuite mise en contact avec un sel de métal alcalino-terreux tel que défini précédemment. Le dépôt de l'oxyde ou hydroxyde correspondant est réalisé par précipitation in situ via l'addition d'une base.

A cet effet, on procède tout d'abord à la solubilisation du sel alcalino-terreux dans la phase liquide de la dispersion de particules de polymère.

Le sel de l'alcalino-terreux est de préférence un halogénure comme le chlorure ou un sulfate. Plus préférentiellement, il s'agit d'un chlorure ou sulfate de calcium et/ou de magnésium.

On augmente le pH de la phase liquide ainsi obtenue par addition d'une base jusqu'à la manifestation du phénomène de précipitation de l'oxyde ou hydroxyde alcalino-terreux.

Conviennent notamment à l'invention les bases de type soude, potasse ou ammoniaque.

Il est clair que la quantité en sel alcalino-terreux mise en oeuvre est directement fonction de l'épaisseur du dépôt en oxyde ou hydroxyde alcalino-terreux souhaité.

A titre indicatif, cette quantité peut varier entre 0,05 % et 3 % en poids exprimée en poids de matière sèche de la dispersion aqueuse, alcoolique ou hydroalcoolique de polymère à traiter.

Le cas échéant, il peut être nécessaire d'ajuster les autres paramètres de cette opération à savoir temps et température par exemple pour privilégier un bon déroulement de la précipitation dudit dépôt inorganique. Ces ajustements relèvent de la compétence de l'homme du métier.

A l'issue de cette opération de dépôt d'une couche intermédiaire, il est ensuite effectué le dépôt du revêtement externe inorganique.

A cet effet, on met en contact dans la phase liquide de la dispersion de particules de polymère organique, contenant la matière active hydrophobe, et recouvertes au moins en partie d'une couche constituée d'au moins un hydroxyde de métal alcalino-terreux, un sel de silicium, aluminium, zirconium et/ou d'un métal de transition soluble dans la phase liquide de ladite dispersion et un agent susceptible de réagir avec ledit sel soluble pour former un précipité constitué de l'oxyde ou de l'hydroxyde correspondant.

Les sels solubles préférentiellement mis en oeuvre sont choisis parmi les silicates d'un métal alcalin, aluminates d'un métal alcalin, les oxychlorures, chlorures, nitrates, sulfates, d'aluminium, de zirconium ou d'un métal de transition tel que défini précédemment.

L'agent précipitant peut être choisi parmi des composés acides ou basiques. A titre indicatif des agents précipitants convenant à l'invention, on peut plus particulièrement citer les acide phosphorique, sulfurique, acétique, les hydroxydes de métaux alcalins, l'ammoniaque et le dioxyde de carbone.

Les quantités en sel soluble et agent précipitant sont généralement ajustées de manière à obtenir un revêtement externe de 1 à 300 nm et de préférence de 5 à 100 nm d'épaisseur.

Cette opération de précipitation est réalisée en maintenant le pH du milieu dans une gamme permettant une précipitation maximale de l'oxyde ou hydroxyde. D'une manière générale, cette gamme de pH se situe entre 8 et 11. D'une manière préférentielle, ce pH est maintenu constant entre 8 et 11.

L'introduction du sel dans la dispersion est réalisée de manière à éviter la sursaturation en sels, du milieu. En d'autres termes, on évite la formation de particules d'aluminium, de silicium, de zirconium, de métal de transition "hors grain".

Ceci est effectué en contrôlant notamment le débit d'introduction dudit sel, ce que l'homme du métier est en mesure de faire en effectuant de simples essais de routine.

La température de précipitation est de préférence elle aussi contrôlée. Elle est généralement comprise entre 20 et 95 °C et de préférence entre 20 et 75 °C.

L'opération de précipitation a lieu plus particulièrement sous agitation.

De plus, le procédé de préparation de suspensions de particules composites est mis en oeuvre à la pression atmosphérique, bien que des pressions supérieures ou inférieures ne soient pas exclues.

D'une manière préférentielle, cette étape est réalisée par mise en oeuvre d'un silicate de métal alcalin et d'un acide comme agent précipitant, tout particulièrement l'acide sulfurique ou le dioxyde de carbone.

Cette opération est de préférence réalisée à une température de 40 à 60 °C, en maintenant le pH à une valeur de 8 à 10.

On effectue ensuite le séchage des particules composites résultantes.

Celui-ci peut avoir lieu directement sur la suspension obtenue.

Il est possible d'effectuer le séchage des particules dudit milieu selon les méthodes classiques, telle que la centrifugation par exemple.

Il est fait remarquer que cette seconde possibilité est particulièrement avantageuse dans le cas où l'on souhaite faire subir aux particules composites un traitement de surface préalable au séchage.

Ce traitement consiste en règle générale à mettre à nouveau en suspension les particules composites puis à introduire dans la suspension, au moins un composé organique, comme l'acide stéarique, les stéarates, les huiles polysiloxanes entre autres.

Ce type de prétraitement permet d'éviter, si nécessaire, l'agglomération des particules composites au cours de cette étape de séchage. Il permet de même de conférer des propriétés particulières aux particules, comme par exemple un caractère hydrophobe. Ce traitement permet aussi de compatibiliser les particules composites avec le milieu dans lequel elles seront par la suite introduites.

Les particules composites obtenues peuvent également subir une étape de mûrissement. Cette opération peut consister en un chauffage desdites particules à une température compatible avec leur stabilité, de préférence entre 40 et 60°C.

Ce mûrissement peut être effectué pendant au moins une heure.

Selon un mode de réalisation particulier de l'invention, la séparation des particules composites du mélange réactionnel et leur séchage se font par atomisation, c'est-à-dire par pulvérisation du mélange dans une atmosphère chaude (spray-drying). L'atomisation peut être réalisée au moyen de tout pulvérisateur connu en soi, par exemple par une buse de pulvérisation du type pomme d'arrosoir ou autre. On peut également utiliser des atomiseurs dits à turbine. Sur les diverses techniques de pulvérisation susceptibles d'être mises en oeuvre dans le précédent procédé, on pourra se référer notamment à l'ouvrage de base de MASTERS intitulé "SPRAY-DRYING" (deuxième édition, 1976, Editions George Godwin - London).

On notera que l'on peut également mettre en oeuvre l'opération d'atomisation-séchage au moyen d'un réacteur "flash", par exemple du type décrit notamment dans les demandes de brevet français n° 2 257 326, 2 419 754 et 2 431 321.

Les particules revendiquées ou obtenues à l'issue du procédé revendiqué sont particulièrement avantageuses pour le conditionnement de matières actives possédant un caractère hydrophobe.

Comme évoqué précédemment, de part leur enveloppe inorganique dont l'adhérence est renforcée au niveau du coeur organique grâce à la présence d'une couche intermédiaire, elles garantissent une meilleure protection de la matière active vis à vis du milieu extérieur. Ainsi encapsulée, la matière active est mieux protégée contre toute modification de températures, pH, radiation UV et en agents chimiques susceptible de se manifester dans le milieu extérieur.

De même, l'encapsulation selon l'invention garantit à l'utilisateur une protection à l'égard de la matière active encapsulée et de ses effets secondaires qui peuvent notamment se traduire sur les plans olfatiques, visuels ou de la corrosion.

Enfin, à travers le choix des constituants du revêtement externe et de la couche intermédiaire et de leurs épaisseurs respectives, il s'avère possible de moduler la diffusion de la matière active contenue dans les particules composites.

La présente invention a également pour objet l'utilisation des particules composites revendiquées ou obtenues selon l'invention en industries alimentaire, cosmétique, phytosanitaire et pharmaceutique.

Elles sont tout particulièrement utile pour l'encapsulation de matières actives en cosmétique.

Les exemples et figure soumis ci-après sont présentés à titre illustratif et non limitatif de la présente invention.

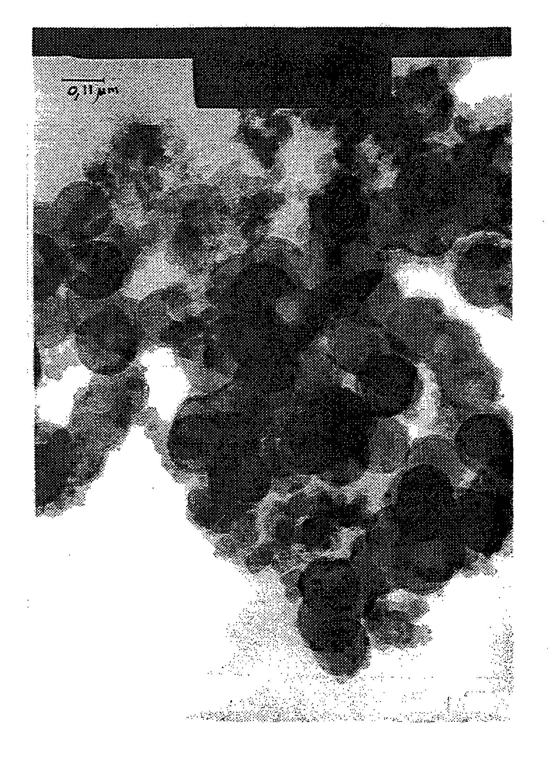

### Figure 1 : Photographie en microscopie électronique en transmission de particules de latex incorporant un agent anti-UV et enrobées d'une écorce minérale. (échelle de 0,11 µm pour 1 cm).

### EXEMPLE 1

### Incorporation d'un agent anti-UV dans un polymère type latex.

Le latex est considéré le Rhodopas SB112®. Il s'agit d'une dispersion aqueuse d'un copolymère styrène-butadiène fonctionnalisé de taille particulaire de 0,19 µm (déterminé par diffusion de la lumière) et possédant un extrait sec de 56 %.

1250 g de polymère, exprimé en poids sec, sont dispersés dans 794 g d'eau, sous agitation à 210 tours/minute dans un réacteur de 4 l et à une température de 85 °C. On y ajoute de façon continue pendant 2 heures, 500 g de Parsol MCX® (2-éthyl hexyl méthoxy cinnamate commercialisé à titre d'anti UV B). A la fin de l'addition, le mélange subit une étape de mûrissement à 85 °C pendant 8 heures. La dispersion est ensuite refroidie à température ambiante et filtrée sur un tamis de 112 µm. Les particules obtenues ont une taille de 0,21 µm.

### EXEMPLE 2

### Préparation de particules de latex comprenant une matière active et enrobées d'un revêtement de silice.

### Matières premières :

- La dispersion de latex préparée selon l'exemple 1
- Chlorure de calcium PROLABO - Normapur
- Tensioactif : Antarox B 848® (alcool polyéthoxylé) (RP-GERRONAZZO®)
- Silicate de sodium (d: 1,33)
- NaOH 1 mole/l
- H₂SO₄ 1 mole/l

| Composition globale : | |
|---|---|
| latex incorporant le Parsol MCX® | 145 g (80 g sec) |
| CaCl₂, 2H₂O | 2,114 g (dissous dans 98 g d'eau) |
| Antarox B848® | 3,2 g pur (solution aqueuse à 10 %) |
| silicate de sodium | 238,88 g |
| eau épurée | 1007 g |
| NaOH 1 mole/l | qsp pH9 |
| H₂SO₄ 1 mole/l | qsp régulation pH 9 (310 g environ) |

### Mode opératoire :

Dans un réacteur de 2 litres, on ajoute le latex modifié et 748 g d'eau épurée. On ajoute ensuite la solution aqueuse d'Antarox B 848® et on agite pendant 1 heure. On ajoute ensuite le chlorure de calcium sous forme de dispersion aqueuse (ajout avec un débit de 4,9 ml/mn). Après l'ajout de chlorure de calcium le pH est ajusté à 9 par de la soude. Le milieu réactionnel est ensuite chauffé à 50 °C. On ajoute après stabilisation de la température et du pH la solution aqueuse de silicate de sodium (débit : 1,6 ml/mn) et la solution d'acide sulfurique (introductions à pH constant). Après l'introduction du silicate on effectue un mûrissement de 2 heures à 50 °C.

Après refroidissement les particules sont séparées par centrifugation, on effectue 3 lavages puis redispersion du gâteau en milieu aqueux.

On observe par microscopie électronique en transmission, MET, un enrobage des particules de latex par une coque de silice de l'ordre d'une dizaine de nanomètres. La figure 1 est une représentation photographique des particules obtenues. Après calcination à 600 °C des particules précédemment obtenues, on obtient des particules creuses de silice de 150 nm environ. Ces résultats confirment bien l'obtention d'une coque inorganique autour d'un coeur organique.

## Revendications

1. Particules composites comprenant au moins
- un coeur composé d'au moins un polymère organique dans lequel est dispersé au moins une matière active hydrophobe, cosmétique, pharmaceutique, alimentaire ou phytosanitaire.
- un revêtement exteme comprenant au moins un oxyde et/ou hydroxyde d'aluminium, de silicium, de zirconium et/ou d'un métal de transition et
- une couche intermédiaire, présente au moins en partie entre le coeur et le revêtement externe et comprenant au moins un hydroxyde de métal alcalino-terreux.

2. Particules composites selon la revendication 1 **caractérisées en ce que** le polymère organique dérive d'un ou plusieurs monomères éthyténiquement insaturés non miscibles à l'eau et polymérisables par voie radicalaire.

3. Particules composites selon la revendication 2 **caractérisées en ce que** les monomères sont choisis parmi les monomères vinylaromatiques, les alkylesters d'acides α-β éthyléniquement insaturés, les esters de vinyle d'acides carboxyliques. les hafogénures de vinyle ou de vinylidène, les diènes aliphatiques conjugués et les nitriles α-β éthyléniquement insaturés.

4. Particules composites selon la revendication 2 ou 3 **caractérisées en ce que** les monomères sont choisis parmi le styréne, butadiène, acrytonilrile, chloroprène, chlorure de vinyle ou de vinylidène, isoprène. isobutylène, acétate de vinyle, propytène. butylène et la vinylpyrrolidone.

5. Particules composites selon l'une des revendications 1 à 4 **caractérisées en ce que** le polymère organique comprend en outre jusqu'à 8 %, de préférence jusqu'à 4 % d'au moins un co-monomère portant des groupes ionogènes polymérisables par voie radicalaire.

6. Particules composites selon la revendication 5 **caractérisées en ce que** le co-monomère portant des groupes ionogènes est choisi parmi les acides carboxyliques α-β éthyléniquement insaturés, les monomères éthyténiques sulfones, les hydroxyalkylesters d'acides α-β éthyléniquement insaturés d'hydroxypropyle, les amides d'acides α-β éthyléniquement insaturés et les amincesters d'acides α-β éthyténiquement insaturés.

7. Particules composites selon la revendication 5 ou 6 **caractérisées en ce que** le co-monomère est choisi parmi les acides acrylique, méthacrylique, itaconique, maléique, crotonique, para-styrènesuifonique, vinylsulfonique, 2-méthacryloyloxyéthylsulfonique, 2-acrylamido-2-méthylpropylsulfonique, vinylbenzène sulfonate, acrylates et méthacrylates d'hydroxyéthyle, acrylamide, méthacrylamide et diéthylaminoéthylméthacrylate.

8. Particules composites selon l'une des revendications précédentes **caractérisées en ce que** le polymère organique constituant le coeur desdites particutes est choisi parmi les polystyrène, copolymères de styrène et d'esters acryliques, de styrène et de butadiène, de styrène, de butadiène et d'acrylamide.

9. Particutes composites selon l'une des revendications précédentes **caractérisées en ce que** le polymère organique possède une température de transition vitreuse comprise entre -100 °C et 150 °C.

10. Particules composites selon la revendication 9 **caractérisées en ce que** la température de transition vitreuse est comprise entre -100 °C et 100 °C.

11. Particules composites selon l'une des revendications précédentes **caractérisées en ce que** le diamètre du coeur en polymère organique incorporant la matière active est compris entre environ 0,03 et 10 µm.

12. Particules composites selon l'une des revendications précédentes **caractérisées en ce que** le revêtement externe est composé d'une seule couche à base d'au moins un oxyde et/ou d'hydroxyde d'aluminium, de silicium, de zirconium et/ou d'un métal de transition.

13. Particules composites selon l'une des revendications 1 à 11 **caractérisées en ce que** le revêtement externe est composé d'au moins deux couches, à base d'au moins un oxyde et/ou hydroxyde d'aluminium, de silicium, de zirconium et/ou d'un métal de transition.

14. Particules composites selon l'une des revendications précédentes **caractérisées en ce que** le revêtement externe comprend un oxyde et/ou un hydroxyde de plusieurs éléments dans une même couche.

15. Particules composites selon l'une des revendications précédentes **caractérisées en ce que** l'oxyde et/ou hydroxyde de métal de transition du revêtement externe est choisi parmi les oxydes et/ou hydroxydes de titane, manganèse, fer, cobalt, nickel et de cuivre.

16. Particules composites selon l'une des revendications précédentes **caractérisées en ce que** le revêtement exteme comprend au moins un oxyde et/ou hydroxyde de silicium, aluminium, titane, ou zirconium.

17. Particules composites selon l'une des revendications précédentes **caractérisées en ce que** la couche intermédiaire est composée d'un ou plusieurs hydroxydes d'alcalino-terreux choisis parmi les hydroxyde de calcium et hydroxyde de magnésium.

18. Particules composites selon l'une des revendications précédentes **caractérisées en ce que** l'épaisseur totale du revêtement appliqué en surface du coeur organique et comprenant une ou plusieurs couches à base d'au moins un oxyde et/ou hydroxyde d'aluminium, de silicium, de zirconium, et/ou d'un métal de transition et une couche d'au moins un hydroxyde de métal alcalino-terreux est au plus de 300 nm.

19. Particules composites selon la revendication 18 **caractérisée en ce que** l'épaisseur de revêtement est au moins de 1 nm.

20. Particules composites selon la revendication 18 ou 19 **caractérisées en ce que** l'épaisseur de revêtement est comprise entre 1 et 300 nm.

21. Particules composites selon la revendication 18, 19 ou 20 **caractérisées en ce que** l'épaisseur de revêtement est comprise entre 5 et 100 nm.

22. Particules composites selon l'une des revendications précédentes **caractérisées en ce que** la matière active hydrophobe dispersée dans le coeur de polymère organique est choisie parmi les agents organiques anti-UV, les huiles essentielles ou thérapeutiques, les parfums et les colorants cosmétiques.

23. Particules composites selon l'une des revendications précédentes **caractérisées en ce que** la matière active est dispersée au sein du polymère organique à raison de 0,1 à 200 % en poids exprimé par rapport au poids en polymère sec.

24. Particules composites selon l'une des revendications précédentes **caractérisées en ce qu'**elles présentent une surface spécifique comprise entre environ 1 et 200 m²/g,

25. Particules composites selon l'une des revendications précédentes **caractérisées en ce que** leur diamètre varie entre environ 0,02 et 6 µm.

26. Procédé utile pour la préparation des particules composites définies selon l'une des revendications 1 à 25 **caractérisé en ce qu'**il comprend au moins:
- l'incorporation d'au moins une matière active hydrophobe dans des particules à base d'au moins un polymère organique, lesdites particules étant en dispersion aqueuse, alcoolique ou hydroalcoolique et le cas échéant formées conjointement à l'incorporation de ladite matière active,
- la solubilisation d'au moins un sel de métal alcalino-terreux dans la phase liquide de la dispersion de particules de polymère et contenant au moins une matière active hydrophobe obtenues selon l'étape précédente,
- l'addition d'une base en quantité suffisante pour précipiter une couche d'oxyde ou d'hydroxyde dudit métal alcalino-terreux à la surface desdites particules,
- la mise en contact, dans la phase liquide de la dispersion de particules obtenue à l'issue de l'étape précédente, d'au moins un sel de silicium, aluminium, zirconium et/ou d'un métal de transition hydrosoluble et d'au moins un agent susceptible de réagir avec ledit sel soluble pour former un précipité constitué de l'oxyde ou de l'hydroxyde correspondant à la surface desdites particules et
- le séchage et la récupération des particules composites ainsi obtenues.

27. Procédé selon la revendication 26 **caractérisé en ce que** les particules à base d'au moins un polymère organique sont obtenues par polymérisation radicalaire en émulsion aqueuse, alcoolique ou hydroalcoolique de monomères éthyléniquement insaturés non miscibles à l'eau et polymérisables.

28. Procédé selon la revendication 27 **caractérisé en ce que** les monomères sont tels que définis en revendications 2 à 7.

29. Procédé selon la revendication 27 ou 28 **caractérisé en ce que** la polymérisation radicalaire est conduite en présence d'au moins un émulsifiant.

30. Procédé selon l'une des revendications 27 à 29 en ce que l'incorporation de la matière active hydrophobe est effectuée conjointement à la polymérisation radicalaire en émulsion des monomères.

31. Procédé selon la revendication 30 **caractérisé en ce que** la matière active hydrophobe est solubilisée dans les monomères, préliminairement à leur mise en émulsion aqueuse, alcoolique ou hydroalcoolique puis à leur polymérisation radicalaire.

32. Procédé selon l'une des revendications 27 à 29 **caractérisé en ce que** la matière active hydrophobe est introduite à l'issue de la polymérisation radicalaire des monomères.

33. Procédé selon la revendication 32 **caractérisé en ce que** l'incorporation de la matière active hydrophobe au sein des particules organiques est effectuée par mise en contact desdites particules organiques en dispersion aqueuse, alcoolique ou hydroalcoolique avec ladite matière active, le cas échéant, solubilisée dans un solvant de transfert.

34. Procédé selon la revendication 33 **caractérisé en ce que** le solvant de transfert est choisi parmi les cétones (méthyléthylcétone, cyclohexanone) les dérivés aromatiques (toluène, éthylbsnzène), dérivés aromatiques chlorés (trichlorobenzène), hydrocarbures allphatiques et cycliques (heptane, dodécane, cyclohexane, décaline..,), dialkyléthers, alcools (propanol, pentanol. cyclohexanol...), esters (acétate d'éthyle, propionate de méthyle, mélange de glutamate/adipate/succinate de méthyle) dérivés chlorés aliphatiques (dichlorométhane) et les silicones.

35. Procédé selon la revendication 33 ou 34 **caractérisé en ce que** l'on procède à l'élimination du solvant de transfert préalablement à l'ajout du ou des sels de métaux alcalino-terreux dont dérive la couche intermédiaire.

36. Procédé selon la revendication 35 **caractérisé en ce que** l'on ajoute à la dispersion aqueuse, alcoolique ou hydroalcoolique obtenue à l'issue de la première étape et avant sa mise en contact avec le ou les sel(s) soluble(s) de métaux alcalino-terreux, un ou plusieurs agent(s) stabilisant(s) de type alkylphénol polyéthoxylé, polyéthylène glycol ou polyvinylpyrrolidone.

37. Procédé selon l'une des revendications 27 à 36 **caractérisé en ce que** le sel d'alcalino-terreux solubilisé en seconde étape du procédé est un chlorure ou sulfate de calcium et/ou de magnésium.

38. Procédé selon l'une des revendications 26 à 37 **caractérisé en ce que** le sel de silicium, aluminium, zirconium et/ou d'un métal de transition solubilisé en quatrième étape du procédé est choisi parmi les silicates d'un métal alcalin, aluminates d'un métal alcalin, les oxychlorures, chlorures, nitrates, sulfates d'aluminium, de zirconium ou d'un métal de transition.

39. Procédé selon l'une des revendications 26 à 38 **caractérisé en ce que** l'agent précipitant mis en oeuvre en quatrième étape du procédé est choisi parmi les acides phosphorique, sulfurique, acétique, les hydroxydes de métaux alcalins, l'ammoniaque et le dioxyde de carbone.

40. Procédé selon l'une des revendications 26 à 39 **caractérisé en ce que** les quantités de sel soluble et d'agent précipitant sont ajustées de manière à obtenir un revêtement externe de 1 à 300 nm d'épaisseur.

41. Procédé selon l'une des revendications 26 à 40 **caractérisé en ce que** l'opération de précipitation de l'oxyde et/ou hydroxyde de silicium, aluminium, zirconium et/ou d'un métal de transition est réalisée en maintenant le pH entre 8 et 11.

42. Procédé selon l'une des revendications 26 à 40 **caractérisé en ce que** la quatrième étape met en oeuvre un silicate de métal alcalin et un acide comme agent précipitant.

43. Procédé selon la revendication 42 **caractérisé en ce que** l'acide est l'acide sulfurique ou le dioxyde de carbone.

44. Procédé selon l'une des revendications 26 à 43 **caractérisé en ce que** l'on fait subir aux particules composites un traitement de surface préalablement à leur séchage.

45. Procédé selon l'une des revendications 26 à 44 **caractérisé en ce que** les particules composites obtenues subissent en outre un mûrissement.

46. Utilisation des particules composites selon l'une des revendications 1 à 25 ou obtenues selon les revendications 26 à 45 en industries alimentaire, cosmétique, phytosanitaire et pharmaceutique.

## Patentansprüche

1. Verbundteilchen, die mindestens
- einen Kern, der aus wenigstens einem organischen Polymer zusammengesetzt ist, in welchem mindestens ein kosmetischer, pharmazeutischer, der Ernährung dienender oder Pflanzen schützender hydrophober Wirkstoff dispergiert ist,
- eine äußere Beschichtung, die mindestens ein Oxid und/oder Hydroxid des Aluminiums, Siliciums, Zirconiums und/oder eines Übergangsmetalls umfasst, und
- eine Zwischenschicht, die wenigstens teilweise zwischen dem Kern und der äußeren Beschichtung vorhanden ist und mindestens ein Erdalkalimetallhydroxid umfasst,
umfassen.

2. Verbundteilchen nach Anspruch 1, **dadurch gekennzeichnet, dass** das organische Polymer von einem oder mehreren ethylenisch ungesättigten Monomeren abgeleitet ist, die mit Wasser unmischbar und radikalisch polymerisierbar sind.

3. Verbundteilchen nach Anspruch 2, **dadurch gekennzeichnet, dass** die Monomeren aus vinylaromatischen, Monomeren, Alkylestern von α,β-ethylenisch ungesättigten Säuren, Carbonsäurevinylestern, Vinyl- bzw. vinylidenhalogeniden, konjugierten aliphatischen Dienen und α,β-ethylenisch ungesättigten Nitrilen ausgewählt sind.

4. Verbundteilchen nach Anspruch 2 oder 3, **dadurch gekennzeichnet, dass** die Monomeren aus Styrol, Butadien, Acrylnitril, Chloropren, Vinyl- bzw. Vinylidenchlorid, Isopren, Isobutylen, Vinylacetat, Propylen, Butylen und Vinylpyrrolidon ausgewählt sind.

5. Verbundteilchen nach einem der Ansprüche 1 bis 4, **dadurch gekennzeichnet, dass** das organische Polymer außerdem bis zu 8 % und vorzugsweise bis zu 4 % mindestens eines Comonomers umfasst, das ionogene Gruppen trägt, die radikalisch polymerisierbar sind.

6. Verbundteilchen nach Anspruch 5, **dadurch gekennzeichnet, dass** das ionogene Gruppen tragende Comonomer aus α,β-ethylenisch ungesättigten Carbonsäuren, sulfonierten ethylenischen Monomeren, Hydroxyalkylestern von α,β-ethylenisch ungesättigten Hydroxypropylsäuren, α,β-ethylenisch ungesättigten Säureamiden und Aminoestern von α,β-ethylenisch ungesättigten Säuren ausgewählt ist.

7. Verbundteilchen nach Anspruch 5 oder 6, **dadurch gekennzeichnet, dass** das Comonomer aus Acryl-, Methacryl-, Itacon-, Malein-, Croton-, p-Styrolsulfon-, Vinylsulfon-, 2-Methacryloyloxyethylsulfon- und 2-Acrylamido-2-methylpropylsulfonsäure, Vinylbenzolsulfonat, Hydroxyethylacrylat und -methacrylat, Acrylamid, Methacrylamid und Diethylaminoethylmethacrylat ausgewählt ist.

8. Verbundteilchen nach einem der vorhergehenden Ansprüche, **dadurch gekennzeichnet, dass** das den Kern dieser Teilchen bildende organische Polymer aus Polystyrol und Copolymeren aus Styrol und Acrylsäureestern, Styrol und Butadien und Styrol, Butadien und Acrylamid ausgewählt ist.

9. Verbundteilchen nach einem der vorhergehenden Ansprüche, **dadurch gekennzeichnet, dass** das organische Polymer eine Glasübergangstemperatur von - 100 bis 150 °C besitzt.

10. Verbundteilchen nach Anspruch 9, **dadurch gekennzeichnet, dass** die Glasübergangstemperatur -100 bis 100 °C beträgt.

11. Verbundteilchen nach einem der vorhergehenden Ansprüche, **dadurch gekennzeichnet, dass** der Durchmesser des Kerns aus organischem Polymer, das den Wirkstoff enthält, etwa 0,03 bis 10 um beträgt.

12. Verbundteilchen nach einem der vorhergehenden Ansprüche, **dadurch gekennzeichnet, dass** die äußere Beschichtung aus einer einzigen Schicht auf der Basis von mindestens einem Oxid und/oder Hydroxid des Aluminiums, Siliciums, Zirconiums und/oder eines Übergangsmetalls zusammengesetzt ist.

13. Verbundteilchen nach einem der Ansprüche 1 bis 11, **dadurch gekennzeichnet, dass** die äußere Beschichtung aus mindestens zwei Schichten auf der Basis mindestens eines Oxids und/oder Hydroxids des Aluminiums, Siliciums, Zirconiums und/oder eines Übergangsmetalls zusammengesetzt ist.

14. Verbundteuchen nach einem der vorhergehenden Ansprüche, **dadurch gekennzeichnet, dass** die äußere Beschichtung ein Oxid und/oder ein Hydroxid von mehreren Elementen in ein und derselben Schicht umfasst.

15. Verbundteilchen nach einem der vorhergehenden Ansprüche, **dadurch gekennzeichnet, dass** das Übergangsmetalloxid und/oder -hydroxid der äußeren Beschichtung aus den Oxiden und/oder Hydroxiden des Titans, Mangans, Eisens, Cobalts, Nickels und Kupfers ausgewählt ist.

16. Verbundteilchen nach einem der vorhergehenden Ansprüche, **dadurch gekennzeichnet, dass** die äußere Beschichtung mindestens ein Oxid und/oder Hydroxid des Siliciums, Aluminiums, Titans oder Zirconiums umfasst.

17. Verbundteilchen nach einem der vorhergehenden Ansprüche, **dadurch gekennzeichnet, dass** die Zwischenschicht aus einem oder mehreren Erdalkalimetallhydroxiden zusammengesetzt ist, das/die aus Calcium- und Magnesiumhydroxid ausgewählt ist/sind.

18. Verbundteilchen nach einem der vorhergehenden Ansprüche, **dadurch gekennzeichnet, dass** die Gesamtdicke der Beschichtung, die auf die Oberfläche des organischen Kerns aufgebracht worden ist und eine oder mehrere Schichten auf der Basis von mindestens einem Oxid und/oder Hydroxid des Aluminiums, Siliciums, Zirconiums und/oder eines Übergangsmetalls und eine Schicht aus mindestens einem Erdalkalimetallhydroxid umfasst, höchstens 300 nm beträgt.

19. Verbundteilchen nach Anspruch 18, **dadurch gekennzeichnet, dass** die Dicke der Beschichtung mindestens 1 nm beträgt.

20. Verbundteilchen nach Anspruch 18 oder 19, **dadurch gekennzeichnet, dass** die Dicke der Beschichtung 1 bis 300 nm beträgt.

21. Verbundteilchen nach Anspruch 18, 19 oder 20, **dadurch gekennzeichnet, dass** die Dicke der Beschichtung 5 bis 100 nm beträgt.

22. Verbundteilchen nach einem der vorhergehenden Ansprüche, **dadurch gekennzeichnet, dass** der hydrophobe Wirkstoff, der im organischen Polymerkern dispergiert ist, aus organischen ÜV-Blockern, therapeutischen oder etherischen Ölen, Parfums und kosmetischen Farbmitteln ausgewählt ist.

23. Verbundteilchen nach einem der vorhergehenden Ansprüche, **dadurch gekennzeichnet, dass** der Wirkstoff im organischen Polymer mit einem Anteil von 0,1 bis 200 Gew.%, bezogen auf das Gewicht des trockenen Polymers, dispergiert ist.

24. Verbundteilchen nach einem der vorhergehenden Ansprüche, **dadurch gekennzeichnet, dass** sie eine spezifische Oberfläche von etwa 1 bis 200 m²/g besitzen.

25. Verbundteilchen nach einem der vorhergehenden Ansprüche, **dadurch gekennzeichnet, dass** ihr Durchmesser zwischen etwa 0,02 und 6 µm variiert.

26. Verfahren, das zur Herstellung der Verbundteilchen nach einem der Anspruche 1 bis 25 nützlich ist, **dadurch gekennzeichnet, dass** es wenigstens das
- Einbauen mindestens eines hydrophoben Wirkstoffs in Teilchen auf der Basis mindestens eines organischen Polymers, die sich in einer wässrigen, alkoholischen oder wässrig alkoholischen Dispersion befinden und gegebenenfalls gleichzeitig mit dem Einbau dieses Wirkstoffs gebildet werden,
- Solubilisieren wenigstens eines Erdalkalimetallsalzes in der Flüssigphase der Dispersion von Polymerteilchenn, die mindestens einen hydrophoben Wirkstoff enthalten und gemäß der vorhergehenden Stufe erhalten worden sind,
- Zugeben einer Base in einer Menge, die ausreicht, um eine Schicht aus dem Oxid oder Hydroxid des Erdalkalimetalls auf der Oberfläche der Teilchen abzuscheiden,
- In-Berührung-Bringen mindestens eines wasserlöslichen Salzes des Siliciums, Aluminiums, Zirconiums und/oder eines Übergangsmetalls mit mindestens einem Agens, das in der Lage ist, mit diesem löslichen Salz zu reagieren, um einen Niederschlag zu bilden, der aus dem Oxid oder Hydroxid besteht, das der Oberfläche der Teilchen entspricht, in der Flüssigphase der Teilchendispersion, die nach der vorhergehenden Stufe erhalten worden ist, und
- Trocknen und Gewinnen der so erhaltenen Verbundteilchen
umfasst.

27. Verfahren nach Anspruch 26, **dadurch gekennzeichnet, dass** die Teilchen auf der Basis mindestens eines organischen Polymers durch radikalische Polymerisation von mit Wasser unmischbaren, polymerisierbaren ethylenisch ungesättigten Monomeren in wässriger, alkoholischer oder wässrig alkoholischer Emulsion erhalten werden.

28. Verfahren nach Anspruch 27, **dadurch gekennzeichnet, dass** die Monomeren wie in den Ansprüchen 2 bis 7 definiert sind.

29. Verfahren nach Anspruch 27 oder 28, **dadurch gekennzeichnet, dass** die radikalische Polymerisation in Gegenwart mindestens eines Emulgators durchgeführt wird.

30. Verfahren nach einem der Ansprüche 27 bis 29, **dadurch gekennzeichnet, dass** der Einbau des hydrophoben Wirkstoffs gleichzeitig mit der radikalischen Emulsionspolymerisation der Monomeren durchgeführt wird.

31. Verfahren nach Anspruch 30, **dadurch gekennzeichnet, dass** der hydrophobe Wirkstoff in den Monomeren solubilisiert wird, bevor diese in eine wässrige, alkoholische oder wässrig alkoholische Emulsion überführt werden, wonach sie radikalisch polymerisiert werden.

32. Verfahren nach einem der Ansprüche 27 bis 29, **dadurch gekennzeichnet, dass** der hydrophobe Wirkstoff nach der radikalischen Polymerisation der Monomeren eingebaut wird.

33. , Verfahren nach Anspruch 32, **dadurch gekennzeichnet, dass** der Einbau des hydrophoben Wirkstoffs in die organischen Teilchen durch deren In-Berührung-Bringen mit dem Wirkstoff, der gegebenenfalls in einem Lösungsvermittler solubilisiert ist, in einer wässrigen, alkoholischen oder wässrig alkoholischen Dispersion erfolgt.

34. Verfahren nach Anspruch 33, **dadurch gekennzeichnet, dass** der Lösungsvermittler aus Ketonen (Methylethylketon, Cyclohexanon), aromatischen Verbindungen (Toluol, Ethylbenzol), chlorierten aromatischen Verbindungen (Trichlorbenzol), aliphatischen und cyclischen Kohlenwasserstoffen (beispielsweise Heptan, Dodecan, Cyclohexan und Decalin), Dialkylethern, Alkoholen (beispielsweise Propanol, Pentanol und Cyclohexanol), Estern (Ethylacetat, Methylpropionat, Methylglutamat/-adipat/-succinat-Gemisch), chlorierten aliphatischen Verbindungen (Dichlormethan) und Siliconen ausgewählt wird.

35. Verfahren nach Anspruch 33 oder 34, **dadurch gekennzeichnet, dass** die Entfernung des Lösungsvermittlers vor der Zugabe des/der Erdalkalimetallsalze/s, von welchem/welchen die Zwischenschicht abgeleitet ist, durchgeführt wird.

36. Verfahren nach Anspruch 35, **dadurch gekennzeichnet, dass** zu der wässrigen, alkoholischen oder wässrig alkoholischen Dispersion, die nach der ersten Stufe erhalten worden ist, und vor deren In-Berührung-Bringen mit dem/den löslichen Erdalkalimetallsalz/en ein oder mehrers Stabilisierungsmittel vom Typ polyethoxyliertes Alkylphenol, Polyethylenglykol oder Polyvinylpyrrolidon zugegeben werden.

37. Verfahren nach einem der Ansprüche 27 bis 36, **dadurch gekennzeichnet, dass** das in der zweiten Stufe des Verfahrens solubilisierte Erdalkalimetallsalz das Chlorid oder Sulfat des Calciums und/oder Magnesiums ist.

38. Verfahren nach einem der Ansprüche 26 bis 37, **dadurch gekennzeichnet, dass** das Salz des Siliciums, Aluminiums, Zirconiums und/oder eines übergangsmetalls, das in der vierten Stufe des Verfahrens solubilisiert worden ist, aus Silicaten eines Alkalimetalls, Aluminaten eines Alkalimetalls, Chloridoxiden, Chloriden, Nitraten und Sulfaten des Aluminiums, Zirconiums oder eines Übergangsmetalls ausgewählt ist.

39. Verfahren nach einem der Ansprüche 26 bis 38, **dadurch gekennzeichnet, dass** das in der vierten Stufe des Verfahrens eingesetzte Fällungsmittel aus Phosphor-, Schwefel- und Essigsäure, Alkalimetallhydroxiden, Ammoniak und Kohlendioxid ausgewählt ist.

40. Verfahren nach einem der Ansprüche 26 bis 39, **dadurch gekennzeichnet, dass** die Menge des löslichen Salzes und die des Pällungsmittels derart eingestellt werden, dass eine 1 bis 300 nm dicke äußere Beschichtung erhalten wird.

41. Verfahren nach einem der Ansprüche 26 bis 40, **dadurch gekennzeichnet, dass** das Ausfällen des Oxids und/oder Hydroxids des Siliciums, Aluminiums, Zirconiums und/oder eines Übergangsmetalls durchgeführt wird, indem der pH-Wert auf zwischen 8 und 11 gehalten wird.

42. Verfahren nach einem der Ansprüche 26 bis 40, **dadurch gekennzeichnet, dass** in der vierten Stufe ein Alkalimetallsilicat und eine Säure als Fällungsmittel eingesetzt werden.

43. Verfahren nach Anspruch 42, **dadurch gekennzeichnet, dass** die Säure Schwefelsäure oder Kohlendioxid ist.

44. Verfahren nach einem der Ansprüche 26 bis 43, **dadurch gekennzeichnet, dass** die Verbundteilchen vor dem Trocknen oberflächenbehandelt werden.

45. Verfahren nach einem der Ansprüche 26 bis 44, **dadurch gekennzeichnet, dass** die erhaltenen Verbundteilchen außerdem altern gelassen werden.

46. Verwendung der Verbundteilchen nach einem der Ansprüche 1 bis 25 oder erhalten nach den Ansprüchen 26 bis 45 in der pharmazeutischen, Lebensmittel-, Kosmetik- und Pflanzenschutzmittelindustrie.

## Claims

1. Composite particles comprising at least
- a core composed of at least an organic polymer in which is dispersed at least an active hydrophobic, cosmetic, pharmaceutical, foodstuff or plant-health material,
- an outer coating comprising at least an oxide and/or hydroxide of aluminium, silicon, zirconium and/or of a transition metal, and
- an intermediate layer, present at least partly between the core and the outer coating and comprising at least an alkaline-earth metal hydroxide.

2. Composite particles according to Claim 1, **characterised in that** the organic polymer derives from one or more ethylenically unsaturated monomers non-miscible with water and radicularly polymerisable.

3. Composite particles according to Claim 2, **characterised in that** the monomers are chosen from among vinyl aromatic monomers, ethelenically unsaturated α-β acid alkyl esters, vinyl esters of carboxylic acids, vinyl or vinylidene halogenides, conjugated aliphatic dienes and ethylenically unsaturated α-β nitriles.

4. Composite particles according to Claim 2 or 3, **characterised in that** the monomers are chosen from among, styrene, butadiene, acrylonitrile, chloroprene, vinyl or vinylidene chloride, isoprene, isobutylene, vinyl acetate, propylene, butylene and vinyl pyrrolidone.

5. Composite particles according to one of the Claims 1 to 4, **characterised in that** the organic polymer includes in addition up to 8%, preferably up to 4%, of at least one co-monomer radicularly carrying polymerisable ionogen groups.

6. Composite particles according to Claim 5, **characterised in that** the co-monomer carrying ionogen groups is chosen from among α-β ethylenically unsaturated carboxylic acids, ethylenic sulphonated monomers, ethylenically unsaturated α-β acid hydroxy alkyl esters of hydroxy propyl, ethylenically unsaturated α-β acid amides and ethylenically unsaturated α-β acid amino esters.

7. Composite particles according to Claim 5 or 6, **characterised in that** the co-monomer is chosen from among acrylic, methacrylic, itaconic, maleic, crotonic, para-styrene sulphonic, vinyl sulphonic, 2-methacryloyl cycloxy ethyl sulphonic, 2-acrylamide-2-methyl propyl sulphonic, vinyl benzene sulphonate, acrylates and methacrylates of hydroxy-ethyl, acrylamide, methacrylamide and diethyl amino ethyl methacrylate acids.

8. Composite particles according to one of the previous claims, **characterised in that** the organic polymer comprising the core of the said particles is chosen from among polystyrene, copolymers of styrene and of acrylic esters, of styrene and butadiene, of styrene, butadiene and acrylamide.

9. Composite particles according to one of the previous claims, **characterised in that** the organic polymer has a vitreous transition temperature between -100°C and 150°C.

10. Composite particles according to Claim 9, **characterised in that** the vitreous transition temperature is between -100°C and 100°C.

11. Composite particles according to one of the previous claims, **characterised in that** the diameter of the organic polymer core incorporating the active material is between approximately 0.03 and 10 µm.

12. Composite particles according to one of the previous claims, **characterised in that** the outer coating consists of a single layer with a base of at least an oxide and/or hydroxide of aluminium, silicon, zirconium and/or of a transition metal.

13. Composite particles according to one of the Claims 1 to 11, **characterised in that** the outer coating is composed of at least two layers, with a base of at least an oxide and/or hydroxide of aluminium, silicon, zirconium and/or of a transition metal.

14. Composite particles according to one of the previous claims, **characterised in that** the outer coating includes an oxide and/or a hydroxide of several elements in the same layer.

15. Composite particles according to one of the previous claims, **characterised in that** the transition metal oxide and/or hydroxide of the outer coating is chosen from among the titanium, manganese, iron, cobalt, nickel and copper oxides and/or hydroxides.

16. Composite particles according to one of the previous claims, **characterised in that** the outer coating includes at least a silicon, aluminium, titanium or zirconium oxide and/or hydroxide.

17. Composite particles according to one of the above claims, **characterised in that** the intermediate layer is composed of one or more alkaline-earth hydroxides chosen from among calcium hydroxide and magnesium hydroxide.

18. Composite particles according to one of the above claims, **characterised in that** the total thickness of the coating applied on the surface of the organic core and including one or more layers with a base of at least an aluminium, silicon, zirconium oxide and/or hydroxide, and/or a transition metal and a layer of at least an alkaline-earth metal hydroxide is at the most 300 nm.

19. Composite particles according to Claim 18, **characterised in that** the coating is at least 1 nm thick.

20. Composite particles according to Claim 18 or 19, **characterised in that** the coating is between 1 and 300 nm thick.

21. Composite particles according to Claim 18, 19 or 20, **characterised in that** the coating is between 5 and 100 nm thick.

22. Composite particles according to one of the above claims, **characterised in that** the hydrophobic active material dispersed in the organic polymer core is chosen from among anti-UV organic agents, essential or therapeutic oils, perfumes and cosmetic colouring agents.

23. Composite particles according to one of the above claims, **characterised in that** the active material is dispersed within the organic polymer at the rate of 0.1 to 200% by weight expressed in relation to the dry polymer weight.

24. Composite particles according to one of the above claims, **characterised in that** they have a specific surface included between around 1 and 200 sq.m/g.

25. Composite particles according to one of the above claims, **characterised in that** their diameter varies between around 0.02 and 6 µm.

26. Process necessary for preparing the composite particles defined according to one of the Claims 1 to 25, **characterised in that** it comprises at least:
- the incorporation of at least one hydrophobic active material with a base of at least one organic polymer, the said particles being in an aqueous, alcoholic or hydro-alcoholic dispersion and, if applicable, formed in conjunction with the incorporation of the said active material;
- solubilising at least an alkaline-earth metal salt in the liquid phase of the dispersion of polymer particles and containing at least one hydrophobic active material obtained according to the previous stage;
- adding a base in sufficient quantity to precipitate a layer of oxide or hydroxide of the said alkaline-earth metal at the surface of the said particles;
- bringing into contact, in the liquid phase of the dispersion of particles achieved at the end of the previous stage, at least one salt of silicon, aluminium, zirconium and/or of a hydrosoluble transition metal, and at least an agent capable of reacting with the said soluble salt to form a precipitate consisting of the corresponding oxide or hydroxide at the surface of the said particles, and
- drying and recovering the composite particles thus produced.

27. Process according to Claim 26, **characterised in that** the particles with a base of at least one organic polymer are obtained by radicular polymerisation in an aqueous, alcoholic or hydro-alcoholic emulsion of ethylenically unsaturated monomers which are non miscible in water and are polymerisable.

28. Process according to Claim 27, **characterised in that** the monomers are as defined in Claims 2 to 7.

29. Process according to Claim 27 or 28, **characterised in that** the radicular polymerisation is carried out in the presence of at least one emulsifying agent.

30. Process according to one of the Claims 27 to 29 **characterised in that** the hydrophobic active material is incorporated jointly with the radicular polymerisation as an emulsion of the monomers.

31. Process according to Claim 30, **characterised in that** the hydrophobic active material is solubilised in monomers prior to their being put into an aqueous, alcoholic or hydro-alcoholic emulsion and then radicularly polymerised.

32. Process according to one of the Claims 27 to 29, **characterised in that** the hydrophobic active material is introduced at the end of the radicular polymerisation of the monomers.

33. Process according to Claim 32, **characterised in that** the hydrophobic active material is incorporated within the organic particles by putting the said organic particles in an aqueous, alcoholic or hydro-alcoholic dispersion in contact with the said active material, where applicable solubilised in a transfer solvent

34. Process according to Claim 33, **characterised in that** the transfer solvent is chosen from among the ketones (methyl ethyl ketone, cyclohexanone), aromatic derivatives (toluene, ethyl benzene), chlorinated aromatic derivatives (trichlorobenzene), aliphatic and cyclic hydrocarbons (heptane, dodecane, cyclohexane, decalin etc.), dialkyl ethers, alcohols (propanol, pentanol, cyclohexanol etc.), esters (ethyl acetate, methyl propionate, mixture of glutamic acid / adipic acid / succinic acid of methyl) chlorinated aliphatic derivatives (dichloro methane) and silicones.

35. Process according to Claim 33 or 34, **characterised in that** the transfer solvent is eliminated prior to adding the alkaline-earth metal salt or salts from which the intermediate layer derives.

36. Process according to Claim 36, **characterised in that** one or more stabilising agent(s) of the polyethoxyl alkyl phenol, polyethylene glycol or polyvinyl pyrrolidone type is/are added to the aqueous, alcoholic or hydro-alcoholic dispersion produced at the end of the first stage and before it is brought into contact with the soluble alkaline-earth metal salt or salts.

37. Process according to one of the Claims 27 to 36, **characterised in that** the alkaline-earth salt solubilised in the second stage of the process is a chloride or sulphate of calcium and/or of magnesium.

38. Process according to one of the Claims 26 to 37, **characterised in that** the silicon, aluminium, zirconium and/or transition metal salt solubilised in the fourth stage of the process is chosen from among the silicates of an alkaline metal, aluminates of an alkaline metal, oxychlorides, chlorides, nitrates, sulphates of aluminium, zirconium or of a transition metal.

39. Process according to one of the Claims 26 to 38, **characterised in that** the precipitating agent used in the fourth stage of the process is chosen from among phosphoric, sulfuric, acetic acids, alkaline metal hydroxides, ammoniac and carbon dioxide

40. Process according to one of the Claims 26 to 39, **characterised in that** the quantities of soluble salt and of precipitating agent are adjusted so as to obtain an outer coating of 1 to 300 nm thick.

41. Process according to one of the Claims 26 to 40, **characterised in that** the operation of precipitating the oxide and/or hydroxide of silicon, aluminium, zirconium and/or of a transition metal is carried out while keeping the pH between 8 and 11.

42. Process according to one of the Claims 26 to 40, **characterised in that** the fourth stage uses an alkaline metal silicate and an acid as the precipitating agent.

43. Process according to Claim 42, **characterised in that** the acid is sulphuric acid or carbon dioxide.

44. Process according to one of the Claims 26 to 43, **characterised in that** the composite particles are subjected to a surface treatment prior to drying them.

45. Process according to one of the Claims 26 to 44, **characterised in that** the composite particles produced are also subjected to maturing.

46. Use of the composite particles according to one of the Claims 1 to 25 or produced according to Claims 26 to 45 in the foodstuff, cosmetic, plant-health and pharmaceutical industries.
